(19) Europäisches Patentamt
European Patent Office
Office européen des brevets

(11) Publication number: 0 225 478 B1

(12) # EUROPEAN PATENT SPECIFICATION

(45) Date of publication of the patent specification: 16.05.90

(51) Int. Cl.⁵: C07C 205/12, C07C 317/14

(21) Application number: 86115223.9

(22) Date of filing: 03.11.86

(54) **Iodonium ylide antimicrobial compounds.**

(30) Priority: 04.11.85 US 794950

(43) Date of publication of application: 16.06.87 Bulletin 87/25

(45) Publication of the grant of the patent: 16.05.90 Bulletin 90/20

(84) Designated Contracting States: BE CH DE FR GB IT LI NL SE

(56) References cited:
EP-A- 0 160 322

CHEMICAL ABSTRACTS, vol. 69, 1968, Columbus, OH (US); B.KARELE et al.: "Iodonium derivatives of beta-dicarbonyl compounds. XI. Synthesis and properties of some nitrophenyliodonium betaines and dimedone and malonic acidesters", p. 247, no. 2626g
JOURNAL OF THE AMERICAN CHEMICAL SOCIETY, vol. 107, no. 5, 1985, pages 1375-1378; R.M. MORIARTY et al.: "Capture of electron-deficient species with aryl halides. New syntheses of hypervalent iodonium ylides"
SYNTHESIS, May 1983, pages 392-395; K. SCHANK et al.: "Ozonolytic fragmentation of phenyliodonium beta-diketonates; a convenient synthesis of unsolvated vic-triketones"

(73) Proprietor: THE DOW CHEMICAL COMPANY, 2030 Dow Center Abbott Road P.O. Box 1967, Midland Michigan 48640-1967(US)

(72) Inventor: Relenyi, Attila G., 516 Bark Lane, Midland, MI 48640(US)
Inventor: Kruper, William J., Jr., 230 Barton Road, Sanford, MI 48657(US)
Inventor: Walter, Richard W., Jr., 26 Lexington Court, Midland, MI 48640(US)
Inventor: Shankar, Ravi B., 2107 Eastlawn, Midland, MI 48640(US)
Inventor: Zelinko, Anthony P., 701 E Carpenter, Midland, MI 48640(US)
Inventor: Koser, Gerald F., 96 Oakhurst Drive, Munroe Falls, OH 44262(US)

(74) Representative: Weickmann, Heinrich, Dipl.-Ing. et al, Patentanwälte Dipl.-Ing. H.Weickmann Dipl.-Phys.Dr. K.Fincke Dipl.-Ing. F.A.Weickmann Dipl.-Chem. B. Huber Dr.-Ing. H. Liska Dipl.-Phys.Dr. J. Prechtel Postfach 860820, D-8000 München 86(DE)

Note: Within nine months from the publication of the mention of the grant of the European patent, any person may give notice to the European Patent Office of opposition to the European patent granted. Notice of opposition shall be filed in a written reasoned statement. It shall not be deemed to have been filed until the opposition fee has been paid (Art. 99(1) European patent convention).

EP 0 225 478 B1

ACTORUM AG

## Description

The present invention is in the field of industrial antimicrobial compounds.

The present invention is directed to novel iodonium ylide compounds that are useful as antimicrobial agents, e.g., as antibacterial, antialgal, and/or antifungal agents. The compounds of the present invention have the formula

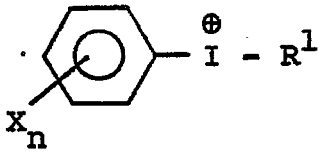

wherein
each X is nitro or

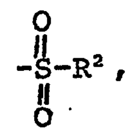

n is 1 or 2, and
$R^1$ represents

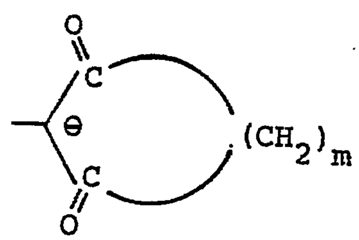

wherein
m is 2 or 3 and
$R^2$ represents
a straight chain alkyl of 1 to 5 carbon atoms,
a branched chain alkyl of 3 to 5 carbon atoms, or
a cyclic alkyl of 3 to 5 carbon atoms, provided that when X is nitro, m is not 3.

In preferred compounds of Formula I, one or two of the X substituents is at the ortho and/or ortho' position, i.e., are substituted at the carbon atoms of the phenyl ring that are adjacent to the carbon atom bonded to the iodine atom.

Preferred X substituents are nitro and

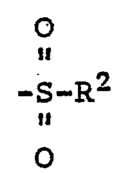

Of the $R^2$ substituents, it is preferred that $R^2$ is methyl.

In the present invention, it is to be noted that all substituent groups are sterically compatible with each other. The term "sterically compatible" is employed to designate substituent groups which are not affected by steric hindrance as this term is defined in "The Condensed Chemical Dictionary", 7th edition, Reinhold Publishing Co., N.Y., page 893 (1966) which definition is as follows:

"steric hindrance. A characteristic
of molecular structure in which
the molecules have a spatial arrangement
of their atoms such that a given
reaction with another molecule is
prevented or retarded in rate."

Sterically compatible may be further defined as reacting compounds having substituents whose physical bulk does not require confinement within volumes insufficient for the exercise of their normal behavior as discussed in "Organic Chemistry" of D. J. Cram and G. Hammond, 2nd edition, McGraw-Hill Book Company, N.Y., page 215 (1964).

The compounds of the present invention are prepared by using procedures known to the art.

The compounds of Formula I can be prepared by reacting the desired substituted diketocyclo compound with the desired X-substituted iodosobenzene in an inert organic solvent such as chloroform (or with an X-substituted iodosobenzene dicarboxylate in basic methanol); this reaction is illustrated by the following reaction sequence:

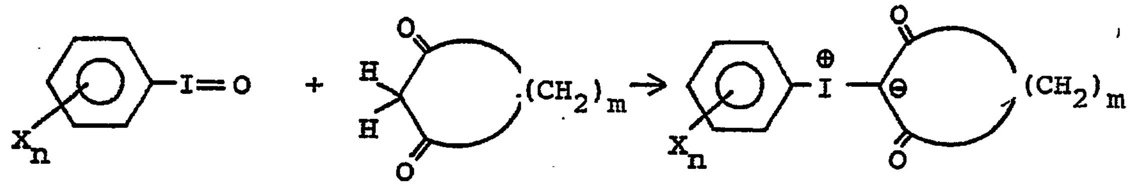

It has heretofore been unknown that the compounds of Formula I can be used in valuable antimicrobial applications (i.e., as a bactericide, fungicide, algaecide and the like). For example, the compounds of Formula I or compositions containing one or more of them as the active antimicrobial constituent can be incorporated into or upon plaster, ink, cosmetic formulations, wallboard, textiles, paper, adhesives, soaps, synthetic detergents, cutting oils, polymeric materials, embalming fluids, oil-base paints, latex paints and any other aqueous based system to prevent the attack of various microbial pests and thus avoid the resultant economic loss due to the degradation of such products by the microorganisms. Also the compounds can be distributed in textiles, cellulosic materials or in grain, or can be employed in the impregnation of wood and lumber to preserve and protect such products from the attack of the organisms of rot, mold and decay.

It is known that antimicrobial agents may alter the digestive process when administered to animals. Therefore, it is contemplated that the compounds of the present invention may have growth promotion effects.

Certain of the compounds of the present invention may exist as complexes, e.g., hydrates and alcoholates. Such complexes are included in the scope of the invention.

The compounds of the present invention are active, to varying degrees, against a variety of bacteria and fungi. As appreciated in the art, the level and spectrum of activity of a specific compound will vary from compound to compound. That is, not all compounds of the present invention will be active against the same organisms at the same concentrations.

Typically, the amount of one or more of the compounds of the present invention applied to target microorganisms or habitat thereof varies from $\leq 1$ to 5,000 parts per million by weight, depending upon the particular compound employed and microorganism treated.

In the protection and preservation of inks, cosmetic formulations, adhesives, soaps, plaster, wallboard, cutting oils, textiles, polymeric materials and paper, good results are obtained when the compounds of the present invention are incorporated in such products in the amount of at least 0.001 percent by weight. In the preservation of wood, excellent results are obtained when the compounds are incorporated, by conventional treatment of the wood, in the amount of at least 0.01 pound per cubic foot (0.16 kg/cubic meter) of wood.

In the preservation and protection of oil and latex paints and primers against destruction caused by the growth of bacteria or fungi, the compounds of the present invention are preferably employed at concentrations of at least 0.001 percent by weight.

In such operations, an effective amount of the unmodified compounds are distributed or incorporated in adhesives, soaps, inks, cosmetic formulations, wallboard, cutting oils, textiles, paper, polymeric materials, paint, lumber, wood products or growth media. However, the compounds of the present invention can also be employed in liquid or dust compositions containing the compounds. In such usage, the compounds are modified with one or a plurality of additaments or adjuvants including water, organic solvents, petroleum oils, petroleum distillates, or other liquid carriers, polymeric thickening agents, urea, surface active dispersing agents and finely divided inert solids. Depending upon the concentration of the compounds used in the compositions, such augmented compositions are adapted to be distributed in inks, adhesives, soaps, cutting oils, polymeric materials, paints, textiles, wallboard, paper, lumber or soil or upon the above-ground surfaces of plants, or to be employed as concentrates and subsequently diluted with additional liquid or solid carriers to produce the ultimate treating compositions. In compositions wherein the adjuvant is a finely divided solid, a surface active agent or the combination of a surface active agent and a liquid diluent, the carrier cooperates with the active component so as to facilitate the invention and to obtain an improved result.

The exact concentration of one or more of the compounds of the present invention to be employed in the treating compositions is not critical and may vary considerably provided the required dosage of the effective agent is supplied in the ink, cosmetic formulation, adhesive, soap, cutting oil, polymeric material, paint, textile, paper, wood or growth medium. The concentration of said compounds in liquid compositions generally is from about 0.0001 to about 3 percent by weight. Concentrations up to 10 percent by weight may be employed. In dusts, the concentrations of the compounds can be from about 0.0001 to

about 95 percent by weight. In compositions to be employed as concentrates, the compounds of the present invention can be present in a concentration of from about 0.01 to about 98 percent by weight. The quantity of treating composition to be applied to textiles, lumber, growth media and the like may vary considerably provided that the required dosage of active ingredients is applied in sufficient amounts of the finished composition to adequately facilitate the penetration and distribution of said ingredients in and on, for example, textiles, lumber and growth media and the like.

In the treatment of lumber, from about 1 to about 100 gallons of solvent composition containing one or more of the compounds of the present invention is usually applied per 1,000 square feet (1 to 100 liters/24.543 square meters) of surface to be treated. In the pressure or vacuum treatment of lumber, sufficient composition is employed adequately to impregnate the wood.

In the preparation of dust compositions, one or more of the compounds of the present invention can be admixed with any of the finely divided solids, such as pyrophyllite,talc, chalk and gypsum and the like. In such operations, the finely divided carrier is ground or mixed with the said compounds or wet with a solution of the compounds in a volatile organic solvent. Similarly, dust compositions containing the products can be prepared using various solid surface active dispersing agents such as fuller's earth, bentonite., attapulgite and other clays. Depending upon the proportions of ingredients, these dust compositions can be employed for the control of pests or employed as concentrates and subsequently diluted with an additional solid surface active dispersing agent or with pyrophyllite, chalk, talc, gypsum and the like to obtain the desired amount of active ingredient in a composition adapted to be employed as described herein. Also, such dust compositions when employed as concentrates can be dispersed in water, with or without the aid of dispersing agents to form spray mixtures.

Further, spray compositions can be prepared by incorporating one or more of the compounds of the present invention, or their liquid or dust concentrate compositions, in mixtures with surface-active dispersing agents such as an ionic or non-ionic emulsifying agent. Such spray compositions are readily employed for the control of microbes or are dispersed in liquid carriers to form diluted sprays containing the compounds in any desired amount suitable for microbial control. The choice of dispersing agents and amounts thereof employed are determined by the ability of the agents to facilitate the dispersion of the concentrate in the liquid carrier to produce the desired spray compositions.

Similarly, the compounds of the present invention can be admixed with a suitable water-immiscible organic liquid and a surface-active dispersing agent to produce an emulsifiable concentrate which can be further diluted with water and oil to form spray mixtures in the form of oil-in-water emulsions. In such compositions, the carrier comprises an aqueous emulsion, i.e., a mixture of water-immiscible solvent, emulsifying agent and water. Preferred dispersing agents which can be employed in these compositions are oil-soluble and include the non-ionic emulsifiers such as the condensation products of alkylene oxides with the inorganic acids, polyoxyethylene derivatives or sorbitan esters, complex ether alcohols and the like. Suitable organic liquids which can be employed in the composition include petroleum oils and distillates, toluene, liquid halohydrocarbons and synthetic organic oils. The surface-active dispersing agents are usually employed in liquid compositions in the amount of from about 0.1 to about 10 to about 20 percent by weight of the combined weight of the dispersing agent and active compound.

The following examples further illustrate the present invention and are not to be construed as a limitation thereon.

Example 1 (2-Methylsulfonyl)-iodosobenzene

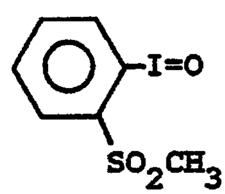

Iodophenyl-2-methyl sulfone (prepared according to the method of H. Zincke and G. Siebert) (1 g 3.54 mmol) was suspended in 5 ml of trifluoro acetic anhydride and cooled to –30°C. To this suspension was added 0.5 ml of concentrated (conc.) $HNO_3$ and the mixture allowed to warm up and stirred at room temperature for about 4 hours. The solvent was then removed under reduced pressure and the resulting solid was dried under high vacuum overnight.

The dried solid was suspended in 25 ml of saturated bicarbonate and stirred overnight. The resulting bright yellow solid was filtered and dried and determined to be the desired product. The yield was 780 mg of desired product having a purity of about 90–95 percent, as determined by iodometric titration.

Example 2 (2-Nitrophenyl)-iodonium 2,5-dioxocyclopentylide

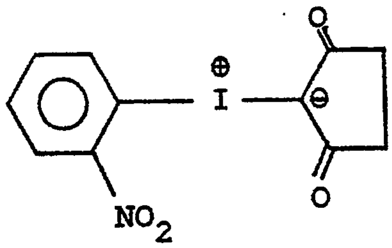

1.73 Grams (0.0176 mol) 1,3-cyclopentanedione and 70 ml acetonitrile were stirred at 22°C for 2 minutes to give a milky white suspension. Subsequent addition of orange iodosonitrobenzene (4.79 g 0.0181 mol) to the milky white mixture, resulted in an immediate color change, and a temperature rise of about 2°C. After 10 minutes, the reaction mixture was filtered to give a white yellow powder (2.8 g 0.0081 mol) (46.1 percent). Approximately 2 g of this powder was dissolved in 200 ml of a 50/50 acetone/methylene chloride mixture and filtered. The yellow filtrate was evaporated at 35° and concentrated to about 10 ml and filtered to give bright yellow crystals. The yellow crystals were dried in vacuo (0.4 mm hg, 25°C, 18 hours) to give 1.00 g 0.0029 mol (23.1 percent) (m.p. 135–140°C) of the desired product which was identified by PMR and CMR (about 95 percent pure).

Analysis

| | C | H | N |
|---|---|---|---|
| Calc. for $C_{11}H_8INO_4$: | 38.29 | 2.33 | 4.06 |
| Found: | 38.2 | 2.47 | 3.90 |

Example 3 ((2-Methylsulfonyl)phenyl)-iodonium 2,6-dioxocyclohexylide

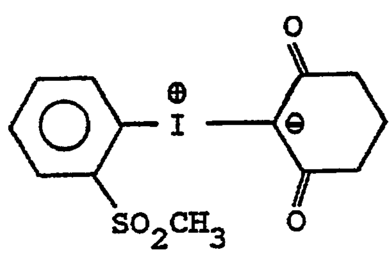

1,3-Cyclohexanedione, (75 mg, 0.67 mmol) was added to a solution of 2-methylsulfonyliodosobenzene (0.67 mmol, 200 mg) in 10 ml of hot methanol. The solution was allowed to cool and was concentrated by blowing nitrogen. The resulting fluffy white solid was filtered and washed with cold methylene chloride dried under vacuum and was determined by NMR to be the desired product: yield 140 mg. More could be obtained by concentration of the filtrate, m.p. 142–143°C (dec).

Analysis

| | C | H |
|---|---|---|
| Calc. for $C_{13}H_{13}IO_4S$: | 39.81 | 3.34 |
| Found: | 39.90 | 3.48 |

The inoculation with bacteria was accomplished using the following procedure. Twenty-four hour cultures of each of the bacteria were prepared by incubating the respective bacteria in tubes containing nutrient broth for 24 hours at 30°C in a shaker. Dilutions of each of the 24 hour cultures were made so that 9 separate suspensions (one for each of the 9 test bacteria) were made, each containing $10^8$ colony forming units (CFU) per ml of suspension of a particular bacteria. Aliquots of 0.3 ml of each of the above suspensions were used to fill individual wells of a Steer's Replicator. For each microbial suspension, 0.3 ml was used to fill 3 wells (i.e., 3 wells of 0.3 ml each) so that for the 9 different bacteria 27 wells were filled. The Steer's Replicator was then used to inoculate the petri plates.

The petri plates were incubated at 30°C for 48 hours and then read to determine if the test compound (i.e., the compound of Example 4) which was incorporated into the agar prevented growth of the respective bacteria. The minimum inhibitory concentration (MIC) for each bacteria was defined as the lowest concentration of the test compound which prevented growth of that bacteria.

The MIC procedure described herein was also performed in nutrient agar at pH 8.2.

Table 1 sets forth the MIC (in ppm) of (2-nitrophenyl)-iodonium 2,6-dioxocyclohexylide for the organisms shown therein at an acid pH (6.8) and at an alkaline pH (8.2).

**TABLE 1**

| | | MIC | |
|---|---|---|---|
| Organism | ATCC No. | Neutral Medium | Alkaline Medium |
| Bacillus subtilis | 8473 | $\leq$1.0 | $\leq$1.0 |
| Enterobacter aerogenes | 13048 | $\leq$1.0 | $\leq$1.0 |
| Escherichia coli | 11229 | $\leq$1.0 | $\leq$1.0 |
| Klebsiella pneumoniae | 8308 | $\leq$1.0 | $\leq$1.0 |
| Proteus vulgaris | 881 | $\leq$1.0 | $\leq$1.0 |
| Pseudomonas aeruginosa | 10145 | $\leq$1.0 | $\leq$1.0 |
| Pseudomonas aeruginosa PRD-10 | 15442 | $\leq$1.0 | $\leq$1.0 |
| Salmonella choleraesuis | 10708 | $\leq$1.0 | $\leq$1.0 |
| Staphylococcus aureus | 6538 | $\leq$1.0 | $\leq$1.0 |

## Claims

1. A compound of the formula

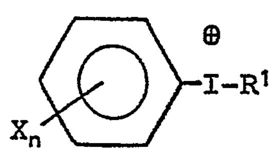

wherein
each X is nitro or

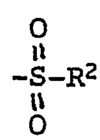 ,

n is 1 or 2,
$R^1$ represents

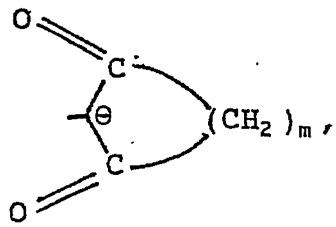

m is 2 or 3, and
$R^2$ represents
a straight chain alkyl of 1 to 5 carbon atoms,
a branched chain alkyl of 3 to 5 carbon atoms, or
a cyclic alkyl of 3 to 5 carbon atoms,
provided that when X is nitro, m is not 3.

2. The compound of Claim 1 wherein X is substituted at one or both of the ortho positions.
3. The compound of Claim 1 or 2 wherein $R^2$ is methyl.
4. The compound of Claim 1 which is (2-nitrophenyl)-iodonium 2,5-dioxocyclopentylide.
5. The compound of Claim 1 which is ((2-methyl-sulfonyl)-phenyl)-iodonium 2,6-dioxocyclohexylide.
6. A method of inhibiting the growth of bacteria, fungi, or algae which comprises contacting said bacteria, fungi, or algae, or habitat thereof, with an effective amount of the compound of any one of Claims 1 to 3.
7. A method of inhibiting the growth of bacteria, fungi, or algae which comprises contacting said bacteria, fungi, or algae, or habitat thereof, with an effective amount of the compound of Claim 4.
8. An antimicrobial composition comprising inert or antimicrobial composition adjuvants in combination with an effective amount of the compound of any one of Claims 1 to 3.
9. An antimicrobial composition comprising inert or antimicrobial composition adjuvants in combination with an effective amount of the compound of Claim 4.

**Patentansprüche**

1. Verbindung der Formel

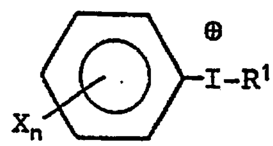

worin jedes X Nitro oder

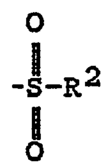

ist,
n 1 oder 2 ist,
$R^1$

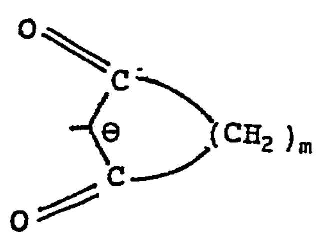

bedeutet,
m 2 oder 3 ist, und
$R^2$ ein geradkettiges Alkyl mit 1 bis 5 Kohlenstoffatomen,
ein verzweigtkettiges Alkyl mit 3 bis 5 Kohlenstoffatomen
oder ein cyclisches Alkyl mit 3 bis 5 Kohlenstoffatomen bedeutet,
unter der Voraussetzung, daß m nicht 3 ist, wenn X Nitro ist.

2. Verbindung nach Anspruch 1, worin X an einer oder an beiden Ortho-Positionen substituiert ist.

3. Verbindung nach Anspruch 1 oder 2, worin $R^2$ Methyl ist.

4. Verbindung nach Anspruch 1, die (2-Nitrophenyl)-Iodonium-2,5-dioxocyclopentylid ist.

5. Verbindung nach Anspruch 1, die ((2-Methylsulfonyl)-phenyl)-Iodonium-2,6-dioxocyclohexylid ist.

6. Verfahren zur Wachstumshemmung von Bakterien, Pilzen oder Algen, umfassend die Behandlung der Bakterien, Pilze oder Algen oder ihres Habitats mit einer wirksamen Menge der Verbindung nach einem der Ansprüche 1 bis 3.

7. Verfahren zur Wachstumshemmung von Bakterien, Pilzen oder Algen, umfassend die Behandlung der Bakterien, Pilze oder Algen oder ihres Habitats mit einer wirksamen Menge der Verbindung nach Anspruch 4.

8. Antimikrobielle Zusammensetzung, die inerte oder antimikrobielle Zusammensetzungs-Hilfsstoffe in Kombination mit einer wirksamen Menge der Verbindung nach einem der Ansprüche 1 bis 3 enthält.

9. Antimikrobielle Zusammensetzung, die inerte oder antimikrobielle Zusammensetzungs-Hilfsstoffe in Kombination mit einer wirksamen Menge der Verbindung nach Anspruch 4 enthält.

## Revendications

1. Composé de formule:

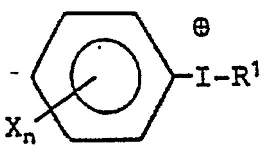

dans laquelle chaque X est nitro ou

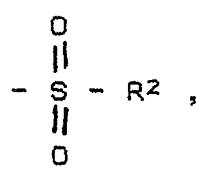

n est 1 ou 2,
$R^1$ représente:

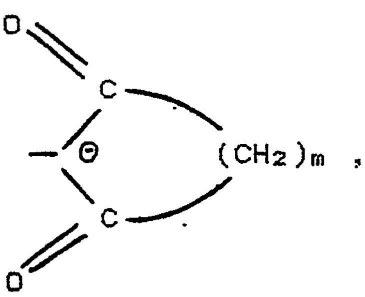

m est 2 ou 3, et
$R^2$ représente un groupe alkyle à chaîne droite ayant 1 à 5 atomes de carbone, un groupe alkyle à chaîne ramifiée ayant 3 à 5 atomes de carbone ou un groupe cycloalkyle ayant 3 à 5 atomes de carbone,
avec la condition que, lorsque X est nitro, m n'est pas 3.

2. Composé selon la revendication 1, dans lequel le substituant X est fixé en l'une des positions ortho ou dans les deux positions ortho.

3. Composé selon la revendication 1 ou 2, dans lequel $R^2$ est méthyle.

4. Composé selon la revendication 1 qui est le 2,5-dioxocyclopentylure de (2-nitrophényl) iodonium.

5. Composé selon la revendication 1 qui est le 2,6-dioxocyclohexylure de ((2-méthylsulfonyl) phényl) iodonium.

6. Procédé d'inhibition de la croissance des bactéries, des champignons ou des algues, qui comprend la mise en contact desdites bactéries, desdits champignons ou desdites algues, ou de leur habitat avec une quantité efficace d'un composé selon l'une quelconque des revendications 1 à 3.

7. Procédé d'inhibition de la croissance des bactéries, des champignons ou des algues, qui comprend la mise en contact desdites bactéries, desdits champignons ou desdites algues, ou de leur habitat avec une quantité efficace du composé selon la revendication 4.

8. Composition anti-microbienne comprenant des additifs de composition inertes ou anti-microbiens en combinaison avec une quantité efficace d'un composé selon l'une quelconque des revendications 1 à 3.

9. Composition anti-microbienne comprenant des additifs de composition inertes ou anti-microbiens en combinaison avec une quantité efficace du composé selon la revendication 4.